# EUROPEAN PATENT SPECIFICATION

(11) **EP 2 968 235 B1**
(45) Date of publication and mention of the grant of the patent: **13.09.2017**
(21) Application number: 14717246.4
(22) Date of filing: 06.03.2014
(51) Int. Cl.: A61K 31/19, A61K 31/7042, A61P 19/06

(54) **COMBINATION OF CANAGLIFLOZIN AND PROBENECID FOR THE TREATMENT OF HYPERURICEMIA**
KOMBINATION AUS CANAGLIFLOZIN UND PROBENECID ZUR BEHANDLUNG VON HYPERURIKÄMIE
COMBINAISON DE CANAGLIFLOZINE ET PROBÉNÉCIDE POUR LE TRAITEMENT DE L'HYPERURICÉMIE

(30) Priority: 15.03.2013 US 201361786738 P
(43) Date of publication of application: 20.01.2016
(73) Proprietor: Janssen Pharmaceutica NV, 2340 Beerse (BE)
(72) Inventor: ROTHENBERG, Paul, Raritan, NJ 08869 (US); WAYS, Douglas., K, Raritan, NJ 08869 (US)
(74) Representative: Carpmaels & Ransford LLP
(86) International application number: PCT/US2014/020958
(87) International publication number: WO 2014/149789

(56) References cited:
- WO-A1-2011/120923
- MUSSO GIOVANNI ET AL: "A novel approach to control hyperglycemia in type 2 diabetes: sodium glucose co-transport (SGLT) inhibitors: systematic review and meta-analysis of randomized trials.", ANNALS OF MEDICINE JUN 2012, vol. 44, no. 4, June 2012 (2012-06), pages 375-393, XP0055098643, ISSN: 1365-2060

## Description

### CROSS REFERENCE TO RELATED APPLICATIONS

This application claims the benefit of U. S. Provisional Application 61/786,738 filed on March 15, 2013.

### FIELD OF THE INVENTION

The present invention is directed to canagliflozin, probenecid, or canagliflozin and probenecid for use in methods for treating hyperuricemia and related disorders, comprising co-therapy with canagliflozin and probenecid.

### BACKGROUND OF THE INVENTION

Hyperuricemia is a condition of high serum total urate levels. In humans and higher primates, uric acid is the final oxidation product of purine catabolism. In most other mammals, however, the enzyme uricase further oxidizes uric acid to allantoin. In human and higher primates, which lack the enzyme uricase, purine metabolites such as xanthine and hypoxanthine are oxidized by xanthine oxidase to uric acid. In human blood, uric acid concentrations between 3.6 mg/dL (∼214/ mol/L) and 8.3 mg/dL (∼494/ mol/L) are considered normal by the American Medical Association. The presence of total urates including uric acid in the serum is important because these compounds are potent antioxidants. In humans, about half the antioxidant capacity of plasma comes from total urates including uric acid.

On the other hand, high serum total urate levels, or hyperuricemia, are often associated with several maladies. For example, high serum total urate levels can lead to a type of arthritis known as gout. Gout is a condition created by a buildup of monosodium urate or uric acid crystals on the articular cartilage of joints, tendons and surrounding tissues due to elevated concentrations of total urate levels in the blood. The build-up of urate or uric acid on these tissues provokes an inflammatory reaction of these tissues. Hyperuricemia is also associated with high or saturating levels of uric acid in urine may result in one form of kidney stones when the uric acid or urate crystallizes in the kidney. These uric acid stones are radiolucent and so do not appear on an abdominal
x-ray. Therefore, their presence must be diagnosed by ultrasound. Some patients with gout eventually develop uric kidney stones.

Additionally, high serum total urate levels are often associated with the so-called metabolic syndrome, including cardiovascular disease and hypertension.

Conventionally, it was believed that high total urate levels are merely innocuous or could even be beneficial because of the antioxidant activity of uric acid. More recently, however, this view has been challenged. Rather, it has been proposed that total urates are an independent risk factor for cardiovascular disease and hypertension. In a rat model, hyperuricemia resulted in lowering endothelial nitric oxide levels, reducing neuronal nitric oxide synthase in the macula densa of the kidney, and stimulating the rennin-angiotensin system. Overtime, the rats developed renal microvascular lesions and eventually hypertension. HEINIG, et al. Cleveland Clinic Journal of Medicine, 2006, pp1059-1064, Vol. 73. Thus, there is evidence that high serum total urate level, or hyperuricemia, is a risk factor for hypertension.

Hyperuricemia is caused either by accelerated generation of total urates and uric acid through purine metabolism or by impaired excretion of total urates in the urine. Consumption of purine-rich diets is one of the causes of hyperuricemia. High levels of fructose in the diet may also cause hyperuricemia. Other dietary causes are ingestion of high protein and fat, and starvation. Starvation results in the body metabolizing its own muscle mass for energy, in the process releasing purines into the bloodstream. Hyperuricemia may lead to renal diseases and may also exacerbate existing renal conditions.

Conventional chronic, prophylactic treatments of gout or other high uric acid-associated diseases include administering to a patient an uricosuric drug, which augments urinary uric acid excretion, such as probenecid, sulfinpyrazone, or benzbromarone; and/or an inhibitor of xanthine oxidase, such as allopurinol, febuxostat, or oxypurinol. A xanthine oxidase inhibitor reduces total urate production in the body. Allopurinol, the most commonly used xanthine oxidase inhibitor, is associated with side-effects in up to 20% of patients. Thus, there remains a need for additional safe and effective treatments for hyperuricemia.

WO 2011/120923 A1 relates to a pharmaceutical composition comprising an SGLT2 inhibitor and a PPARγ agonist.

### SUMMARY OF THE INVENTION

The present invention is directed towards canagliflozin for use in a method for treating hyperuricemia or a related disorder, wherein the method comprises administering to a subject in need thereof, a therapeutically effective amount of co-therapy comprising canagliflozin and probenecid, wherein the term "hyperuricemia or a related disorder" is defined as any disease, disorder or condition characterized by elevated (i.e. above normal) levels serum uric acid.

The present invention is also directed towards probenecid for use in a method for treating hyperuricemia or a related disorder, wherein the method comprises administering to a subject in need thereof, a therapeutically effective amount of co-therapy comprising canaglifozin and probenecid, wherein the term "hyperuricemia or a related disorder" is defined as any disease, disorder or condition characterized by elevated (i.e. above normal) levels serum uric acid.

The present invention is also directed towards canagliflozin and probenecid for use in a method for treating hyperuricemia or a related disorder, wherein the method comprises administering to a subject in need thereof, a therapeutically effective amount of co-therapy comprising canaglifozin and probenecid, wherein the term "hyperuricemia or a related disorder" is defined as any disease, disorder or condition characterized by elevated (i.e. above normal) levels serum uric acid.

In another embodiment, the present invention is directed to canagliflozin and probenecid for use as discussed above, wherein the treatment prevents symptoms of gout. In another embodiment, the present invention is directed to canagliflozin and probenecid for use as discussed above, wherein the hyperuricemia or related disorder is selected from the group consisting of gout, urate nephropathy, chronic kidney disease, hypertension due to hyperurecemia, and kidney stones. In another embodiment, the present invention is directed to canagliflozin and probenecid for use as discussed above, wherein the hyperuricemia or related disorder is selected from the group consisting of gout, urate nephropathy, chronic kidney disease, hypertension due to hyperurecemia, and kidney stones.

In an embodiment, the present invention is directed to canagliflozin for use in a method for the treatment of gout, comprising administering to a subject in need thereof, a therapeutically effective amount of co-therapy comprising canagliflozin and probenecid. In an embodiment, the present invention is directed to probenecid for use in a method for treating gout, wherein the method comprises administering to a subject in need thereof, a therapeutically effective amount of co-therapy comprising canaglifozin and probenecid. In an embodiment, the present invention is directed to canagliflozin and probenecid for use in a method for treating gout, wherein the method comprises administering to a subject in need thereof, a therapeutically effective amount of co-therapy comprising canaglifozin and probenecid.

In a further embodiment, the present invention is directed to a pharmaceutical composition comprising (a) canagliflozin, (b) probenecid and (c) a pharmaceutically acceptable carrier. An illustration of the invention is a pharmaceutical composition made by mixing (a) canagliflozin, (b) probenecid and (c) a pharmaceutically acceptable carrier. Disclosed is a process for making a pharmaceutical composition comprising mixing (a) canagliflozin, (b) probenecid and (c) a pharmaceutically acceptable carrier.

Disclosed is canagliflozin in combination with probenecid for use as a medicament.

Also disclosed is the use of canagliflozin in combination with probenecid in the preparation of a medicament for treating: (a) hyperuicemia, (b) gout, (c) urate nephropathy, (d) chronic kidney disease, (e) hypertension, or (f) kidneys stones in a subject in need thereof.

### BRIEF DESCRIPTION OF THE FIGURES

Figure 1 illustrates measured mean serum urate levels on dosing with canagliflozin alone and in combination with probenecid.
Figure 2 illustrates measured mean urinary uric acid levels on dosing with canagliflozin alone and in combination with probenecid.

### DETAILED DESCRIPTION OF THE INVENTION

The scope of the present invention is defined by the appended claims.

The present invention is directed to canagliflozin, probenecid, or canagliflozin and probenecid for use in a method for the treatment of hyperuricemia or a related disorder, as defined in the claims, comprising administering to a subject in need thereof a therapeutically effective amount of a co-therapy comprising canagliflozin and probenecid.

Also disclosed is canagliflozin, probenecid, or canagliflozin and probenecid for use in methods of lowering serum total urate (uric acid) levels, comprising administering to a
subject in need thereof, a therapeutically effective amount of co-therapy comprising canagliflozin and probenecid.

In another embodiment, the present invention is directed to canagliflozin, probenecid, or canagliflozin and probenecid for use in a method for the treatment of gout (due to hyperuricemia), hypertension (due to hyperuricemia) or urate nephropathy or kidney stones (due to hyperuricemia), comprising administering to a subject in need thereof a therapeutically effective amount of co-therapy comprising canagliflozin and probenecid.

In certain preferred embodiments the present invention is further directed to canagliflozin, probenecid, or canagliflozin and probenecid for use in a method for the treatment of gout comprising administering to a subject in need thereof a therapeutically effective amount of co-therapy comprising canagliflozin and probenecid. Also disclosed is canagliflozin, probenecid, or canagliflozin and probenecid for use in a method for lowering serum uric acid levels or lowering serum total urate, comprising administering to a subject in need thereof a therapeutically effective amount of co-therapy comprising canagliflozin and probenecid.

In another embodiment, the present invention is directed to canagliflozin, probenecid, or canagliflozin and probenecid for use in a method for the treatment of hyperuricemia or a related disorder, as claimed wherein the subject in need thereof is diabetic (preferably, the subject in need thereof is also suffering from TYPE II diabetes mellitus or Syndrome X). In another embodiment, the present invention is directed to canagliflozin, probenecid, or canagliflozin and probenecid for use in a method for the treatment of hyperuricemia or a related disorder, as claimed, wherein the subject in need thereof is non-diabetic.

In another embodiment the present invention is directed to a pharmaceutical composition comprising canagliflozin and probenecid; and a pharmaceutically acceptable carrier; wherein the canagliflozin is in an amount in the range of from about 50 to about 500 mg, preferably in an amount in the range of from about 100 mg to about 300 mg.

In another embodiment the present invention is directed to a pharmaceutical composition comprising canagliflozin and probenecid; and a pharmaceutically acceptable carrier, wherein the probenecid is in an amount in the range of from about 250 to about 1000 mg.

As used herein, unless otherwise noted, the term "canagliflozin" shall mean a compound of formula (I-X) 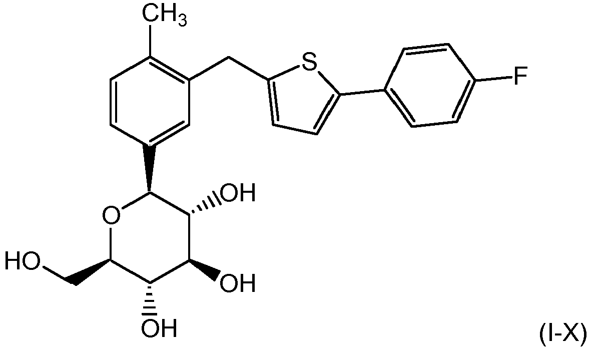 or a crystalline hemihydrate form of the compound of formula (I-X).

The compound of formula (I-X) exhibits inhibitory activity against sodium-dependent glucose transporter, such as for example SGLT2; and may be prepared according to the process as disclosed in Nomura, S. et al., US Patent Publication, US 2005/0233988 A1, published October 20, 2005.

As used herein, the term "canagliflozin" shall further include a mixture of stereoisomers, or each pure or substantially pure isomer. In addition, the term "canagliflozin" shall include an intramolecular salt, hydrate, solvate or polymorph thereof.

In an embodiment, the term "canagliflozin" shall mean the crystalline hemihydrate form of the compound of formula (I-X), as described in WO 2008/069327.

In an embodiment, the present invention is directed to canagliflozin, probenecid, or canagliflozin and probenecid for use in methods for the treatment of hyperuricemia and related disorders, wherein the probenecid is present at a dosage amount in the range of from about 10 mg to about 1000 mg, preferably from about 25 mg to about 500 mg, or any amount or range therein. In another embodiment, the present invention is directed to canagliflozin, probenecid, or canagliflozin and probenecid for use in methods for the treatment of hyperuricemia and related disorders, wherein the canagliflozin is present at a dosage amount in the range of from about 25 mg to about 300 mg, preferably selected from the group consisting of about 50 mg, about 75 mg, about 100 mg, about 150 mg, about 200 mg, about 300 mg and about 500 mg.

In an embodiment, the present invention is directed to canagliflozin, probenecid, or canagliflozin and probenecid for use in methods for the treatment of hyperuricemia and related disorders, wherein the probenecid is administered in an amount in the range of from about 250 mg to about 1000 mg, or any amount or range therein. In another embodiment, the present invention is directed to canagliflozin, probenecid, or canagliflozin and probenecid for use in methods for the treatment of hyperuricemia and related disorders, wherein the canagliflozin is administered in an amount in the range of from about 50 mg to about 500 mg, preferably from about 100 mg to about 300mg.

**Probenecid,** also known as 4-[(dipropylamino)sulfonyl]benzoic acid, is a uricosuric and renal tubular transport blocking agent. Probenecid tablets are indicated for the treatment of the hyperuricemia associated with gout and gouty arthritis.

Probenecid is a uricosuric and renal tubular blocking agent. It inhibits the tubular reabsorption of urate, thus increasing the urinary excretion of uric acid and decreasing serum urate levels. Effective uricosuria reduces the miscible urate pool, retards urate deposition, and promotes resorption of urate deposits. Probenecid inhibits the tubular secretion of penicillin and usually increases penicillin plasma levels by any route the antibiotic is given. A 2-fold to 4-fold elevation has been demonstrated for various penicillins. Probenecid also has been reported to inhibit the renal transport of many other compounds including aminohippuric acid (PAH), aminosalicylic acid (PAS), indomethacin, sodium iodomethamate and related iodinated organic acids, 17-ketosteroids, pantothenic acid, phenolsulfonphthalein (PSP), sulfonamides, and sulfonylureas. Probenecid decreases both hepatic and renal excretion of sulfobromophthalein (BSP). The tubular reabsorption of phosphorus is inhibited in hypoparathyroid but not in euparathyroid individuals. Probenecid does not influence plasma concentrations of salicylates, nor the excretion of streptomycin, chloramphenicol, chlortetracycline, oxytetracycline, or neomycin.

For the treatment of gout, probenecid therapy typically should not be started until an acute gouty attack has subsided. However, if an acute attack is precipitated during therapy, probenecid may be continued without changing the dosage, and full therapeutic dosage of colchicine, or other appropriate therapy, typically is also given to control the acute attack. The recommended adult dosage is 250 mg (e.g., 1/2 probenecid tablet), twice a day for one week, followed by 500 mg (1 tablet) twice a day thereafter. Some degree of renal impairment may be present in patients with gout. A daily dosage of 1000 mg may be adequate. However, if necessary, the daily dosage may be increased by 500 mg increments every 4 weeks within tolerance (and usually not above 2000 mg per day) if symptoms of gouty arthritis are not controlled. Probenecid may not be effective in chronic renal insufficiency particularly when the glomerular filtration rate is 30 mL/minute or less. Probenecid should be continued at the dosage that will maintain normal serum urate levels. When acute attacks have been absent for 6 months or more and serum urate levels remain within normal limits, the daily dosage may be decreased by 500 mg every 6 months. The maintenance dosage should not be reduced to the point where serum urate levels tend to rise.

In an embodiment, the present invention is directed to canagliflozin, probenecid, or canagliflozin and probenecid for use in methods for the treatment of hyperuricemia and related disorders as described herein, wherein probenecid is present at a dosage amount in the range of from about 10 mg to about 1000 mg, preferably from about 50 mg to about 500 mg, preferably from about 250 mg to about 500 mg, or any amount or range therein and canagliflozin is present at a dosage amount in the range of from about 25 mg to about 300 mg, preferably selected from the group consisting of about 50 mg, about 75 mg, about 100 mg, about 150 mg, about 200 mg, about 300 mg and about 500 mg.

In certain embodiments, the present invention is directed to canagliflozin, probenecid, or canagliflozin and probenecid for use in methods for the treatment of hyperuricemia and related disorders, comprising administering to a subject in need thereof co-therapy comprising canagliflozin and probenecid, wherein the therapeutically effective amount of co-therapy is sufficient to treat the hyperuricemia or related disorder without inducing hypouricemia.

In certain embodiments, canagliflozin, probenecid, or canagliflozin and probenecid for use in the methods of the present invention are directed to the treatment of hyperuricemia and related disorders in non-diabetic, as well as diabetic patients. Preferably, the therapeutically effective amount of co-therapy in the methods of the present invention will not cause hypoglycemia in the diabetic and / or non-diabetic patients (more particularly, will not disturb the patient's plasma glucose homeostasis).

In certain embodiments, other uricosurics such as e,g., benzbromarone or sulfinpyrazone may be used in place of probenecid in combination with canagliflozin for the treatment of hyperuricemia and related disorders in a subject in need thereof in accordance with the present invention.

As used herein, unless otherwise noted, the term "**hyperuricemia or a related disorder**" shall include any disease, disorder or condition characterized by elevated (i.e. above normal) levels serum uric acid. Suitably examples include, but are not limited to gout, urate nephropathy, chronic kidney disease, hypertension, and kidney stones. Preferably, the hyperuricemia or related disorder" is selected from the group consisting of gout, urate nephropathy, chronic kidney disease, hypertension, and kidney stones.

As used herein, the terms "**Syndrome X**"**,** "**Metabolic Syndrome**" and "**Metabolic Syndrome X**" shall mean a disorder that presents risk factors for the development of Type 2 diabetes mellitus and cardiovascular disease and is characterized by insulin resistance and hyperinsulinemia and may be accompanied by one or more of the following: (a) glucose intolerance, (b) Type II diabetes mellitus, (c) dyslipidemia, (d) hypertension and (e) obesity.

The term "**subject**" as used herein, refers to an animal, preferably a mammal, most preferably a human, who has been the object of treatment, observation or experiment. Preferably, the subject has experienced and / or exhibited at least one symptom of the disease or disorder to be treated and / or prevented.

As used herein, unless otherwise noted, the terms "**treating**", "**treatment**" and the like, shall include the management and care of a subject or patient (preferably mammal, more preferably human) for the purpose of combating a disease, condition, or disorder and includes the administration of a compound of the present invention to prevent the onset of the symptoms or complications, alleviate the symptoms or complications, or eliminate the disease, condition, or disorder.

As used herein, unless otherwise noted, the term "**prevention**" shall include (a) reduction in the frequency of one or more symptoms; (b) reduction in the severity of one or more symptoms; (c) the delay or avoidance of the development of additional symptoms; and / or (d) delay or avoidance of the development of the disorder or condition.

One skilled in the art will recognize that wherein the present invention is directed to canagliflozin, probenecid, or canagliflozin and probenecid for use in methods of prevention, a subject in need of thereof (i.e. a subject in need of prevention) shall include any subject or patient (preferably a mammal, more preferably a human) who has experienced or exhibited at least one symptom of the disorder, disease or condition to be prevented. Further, a subject in need thereof may additionally be a subject (preferably a mammal, more preferably a human) who has not exhibited any symptoms of the disorder, disease or condition to be prevented, but who has been deemed by a physician, clinician or other medical profession to be at risk of developing said disorder, disease or condition. For example, the subject may be deemed at risk of developing a disorder, disease or condition (and therefore in need of prevention or preventive treatment) as a consequence of the subject's medical history, including, but not limited to, family history, pre-disposition, co-existing (comorbid) disorders or conditions, genetic testing, and the like.

The term "**therapeutically effective amount**" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to co-therapy or combination therapy, comprising administration of (a) canagliflozin and (b) probenecid, "**therapeutically effective amount**" shall mean that amount of the combination of agents taken together so that the combined effect elicits the desired biological or medicinal response. For example, the therapeutically effective amount of co-therapy comprising administration of (a) canagliflozin and (b) probenecid, would be the amount of (a) canagliflozin and (b) probenecid that when taken together or sequentially have a combined effect that is therapeutically effective. Further, it will be recognized by one skilled in the art that in the case of co-therapy with a therapeutically effective amount, as in the example above, the amount of the (a) canagliflozin and / or the amount of the (b) probenecid individually may or may not be therapeutically effective.

Optimal dosages (for canagliflozin, probenecid and / or co-therapy comprising canagliflozin and probenecid) to be administered may be readily determined by those skilled in the art, and will vary with for example, the mode of administration, the strength of the preparation, and the advancement of the disease condition. In addition, factors associated with the particular patient being treated, including patient age, weight, diet and time of administration, will result in the need to adjust dosages.

As used herein, the term "**composition**" is intended to encompass a product comprising the specified ingredients in the specified amounts, as well as any product which results, directly or indirectly, from combinations of the specified ingredients in the specified amounts.

To provide a more concise description, some of the quantitative expressions given herein are not qualified with the term "**about**". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value. Further, to provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

The present invention further comprises pharmaceutical compositions containing (a) canagliflozin, (b) probenecid and one or more pharmaceutically acceptable carrier(s). Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, from about 10 mg to about 1000 mg of probenecid, preferably from about 25 mg to about 500 mg of probenecid, or any amount or range therein (preferably selected from the group consisting of about 125 mg, about 250 mg, about 500 mg and about 1000 mg of probenecid) and from about 25 mg to about 500 mg of canagliflozin or any amount or range therein (preferably selected from the group consisting of about 50 mg, about 75 mg, about 100 mg, about 150 mg, about 200 mg, and about 300 mg of canagliflozin. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. For preparing solid compositions such as tablets, the principal active ingredient are mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. In certain embodiments, the two active ingredients can be formulated together, e.g., in a bi-layer tablet formulation. When referring to these preformulation compositions as homogeneous, it is meant that the active ingredients are dispersed evenly throughout the composition so that the composition may be readily subdivided into equally effective dosage forms such as tablets, pills and capsules. This solid preformulation composition is then subdivided into unit dosage forms of the type described above containing from about 10 mg to about 1000 mg of probenecid, preferably from about 25 mg to about 500 mg of probenecid, or any amount or range therein and from about 25 mg to about 500 mg of canagliflozin or any amount or range therein. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate. In certain embodiments the outer dosage component and the inner dosage component can include different active ingredients (e.g., the outer can include canagliflozin and the inner can include probenecid, the outer can include probenecid and the inner can include canagliflozin, and the like).

The liquid forms in which the compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

A pharmaceutical composition comprising any of the compounds as defined herein and a pharmaceutically acceptable carrier may be used for use in the method of treating hyperuricemia and related disorders described in the present invention. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

To prepare a pharmaceutical composition of the present invention, canagliflozin and probenecid, as the active ingredients, may be intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse System, Volumes 1-2, edited by Lieberman et al; published by Marcel Dekker, Inc.

Compounds of this invention may be administered in any of the foregoing compositions and according to dosage regimens established in the art whenever treatment of hyperuricemia or a related disorder is required.

For oral administration, the compositions are preferably provided in the form of tablets containing, about 25, about 50, about 100, about 150, about 200, about 250, about 300 or about 500 milligrams of canagliflozin and about 50, about 125, about 250, about 500, or about 1000 milligrams of probenecid. The tablets may be administered on a regimen of 1 to 4 times per day, preferably 1 or 2 times per day.

The following Examples are set forth to aid in the understanding of the invention, and are not intended and should not be construed to limit in any way the invention set forth in the claims which follow thereafter.

### Example 1: Effect of Canagliflozin and Probenecid on Urate Levels in Urine and Serum - Clinical Trial Results

A single-center, open-label, fixed-sequence study to assess the effects of multiple-dose probenecid on multiple-dose of canagliflozin, in healthy subjects, was completed as described below (study NCT01428284) on clinicaltrials.gov registry website). The study consisted of 3 phases: (1) a Screening Phase of approximately 19 days (Day -21 to Day -3), (2) an Open-label Treatment Phase of 20 days (Day -2 to Day 18), and (3) a Follow-up phase (7 to 10 days after discharge on Day 18). The total duration of the study was approximately 49 days.

### Study Patients:

Approximately 14 healthy men and women between 18 and 55 years of age (inclusive), who had a BMI between 18 and 30 kg/m² (inclusive) and body weight of not less than 50 kg, were eligible for enrollment in this study. Subjects with history of (or current) any of the following medical conditions were excluded: (a) Acute or chronic renal insufficiency (eGFR below 90 mL/min/1.73 m²); (b) Kidney or bladder stones (nephrolithiasis); (c) Hyperuricosuria (>800 mg/day), or gout; and / or (d) Hyperuricemia (>6.8 mg/dL, 404 µmol/L). Reasons for subject withdrawal from the study could include the following: (a) Lost to follow-up; (b) Withdrawal of consent; (c) Withdrawal of consent for pharmacogenomic research (Part 1); (d) Subject was not in compliance with requirements of the study and prohibitions and restrictions; and / or (e) A subject could be discontinued from study treatment (final assessments were to be obtained) if the investigator believed that for safety reasons (eg, adverse event) it was in the best interest of the subject to stop study treatment.

A total of 14 subjects were enrolled, out of which 3 subjects were prematurely withdrawn from the study. Eleven subjects completed the study as planned. The majority of subjects were men (13 men and 1 woman), with a median age of 27 years, mean body weight of 79.6 kg, and mean BMI of 25.4 kg/m².

### Study Drugs:

Canagliflozin was supplied as a 300-mg, capsule-shaped, film-coated white tablet oral, debossed with "CFZ" on one side and "300" on the other side (Lot No.: 1DG4510-X; Expiration date: November 013).

Probenecid was supplied as United States Pharmacopeia (USP) 500 mg tablet from a single lot (Lot No.: 394148A; Expiration date: January 2013).

### Dosage and Administration:

All study drugs were taken between 7:30 and 9:30 a.m. with 240 mL of noncarbonated water. Study drug was swallowed whole and not chewed, divided, dissolved or crushed. Subjects remained upright (standing or sitting) and did not lie down for the first 4 hours after morning study drug administration on Day 14 and Day 17. For each subject, on days when both canagliflozin and probenecid were administered, both doses were administered at approximately the same time.

Canagliflozin was administered as a single 300-mg tablet on Days 1 to Day 17. Probenecid was administered b.i.d. as one 500-mg tablet from Day 15 to Day 17. On Day 14 and Day 17, subjects received study drug under fasted conditions, and standardized lunch approximately 4 hours after study drug administration. On all other study days, subjects received canagliflozin and/or probenecid 1 hour before they received a standardized meal. The study diet was standardized by the site dietician in order to minimize the effect on uric acid levels. Subjects were also advised not to consume high purine-enriched foods from screening to completion of the study.

### Clinical Laboratory Tests

All laboratory testing was conducted by a licensed clinical laboratory (Physicians Reference Laboratory [PRL]), 7800 West 110th Street, Overland Park, KS 66210-2304). Clinical laboratory tests included, among others:
(a) Serum chemistry: glucose, creatinine, blood urea nitrogen (BUN), total protein, total bilirubin, phosphate, albumin, calcium, fasting serum uric acid, sodium, potassium, chloride, magnesium, lactic acid dehydrogenase, alkaline phosphatase, alanine transaminase, aspartate transaminase, gamma-glutamyltransferase, bicarbonate, creatine phosphokinase, total cholesterol (screening only), and triglycerides (screening only); and
(b) Urinary uric acid excretion: A 24-hour total urine was collected at the time points specified in the Time and Events Schedule of the study protocol. The samples were mixed thoroughly and stored between 2°C and 8°C until shipment.

Laboratory data were summarized by type of laboratory test. Normal reference ranges and abnormal results were used in the summary of laboratory data. Descriptive statistics were calculated for each laboratory analyte at vaseline, Day 18, and end-of-study.

### RESULTS

### Serum Urate Levels:

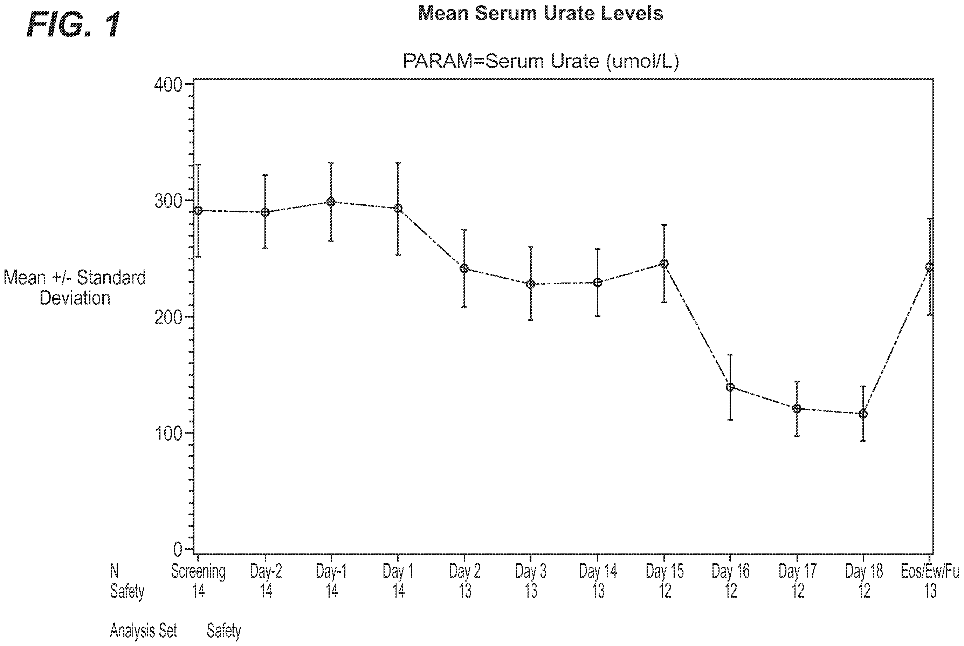

Table 1, below lists mean serum urate levels and calculated mean decrease through the course of the study. At baseline, all patients tested exhibited serum urate levels within the clinically normal range. Figure 1 illustrates said mean serum urate levels (± standard deviation) as a function of study day.

**Table 1: Serum Urate Levels (mmol /d) - 14 patients**

| | **Mean (µmol / L)** | **Mean Decrease (µmol / L)** |
|---|---|---|
| **Screen** | 291.452 | |
| **Day - 2** | 290.177 | |
| **Day - 1** | 298.675 | |

| | **Mean (µmol / L)** | **Mean Decrease (µmol / L)** |
|---|---|---|
| **Day 1** | 293.151 | -5.523 |
| **Day 2** | 241.580 | -57.650 |
| **Day 3** | 228.769 | -70.461 |
| **Day 14** | 229.684 | -69.546 |
| **Day 15** | 245.851 | -52.541 |
| **Day 16** | 139.778 | -158.613 |
| **Day 17** | 121.438 | -176.953 |
| **Day 18** | 116.977 | -181.414 |
| **Follow-up visit** | 242.953 | -52.617 |

Mean serum urate levels (µmol/L) decreased from baseline after administration of canagliflozin alone (by approximately 19%, 24%, and 23%, on Days 2, 3, and 14, respectively). After co-administration of probenecid, further decreases from baseline were observed (approximately 18%, 53%, 59%, and 61% on Days 15, 16, 17, and 18, respectively). At the end-of-study visit (about 7-10 days after the last dose of study drug, mean serum urate levels were about 18% lower compared to baseline.

### Urine Uric Acid Excretion:

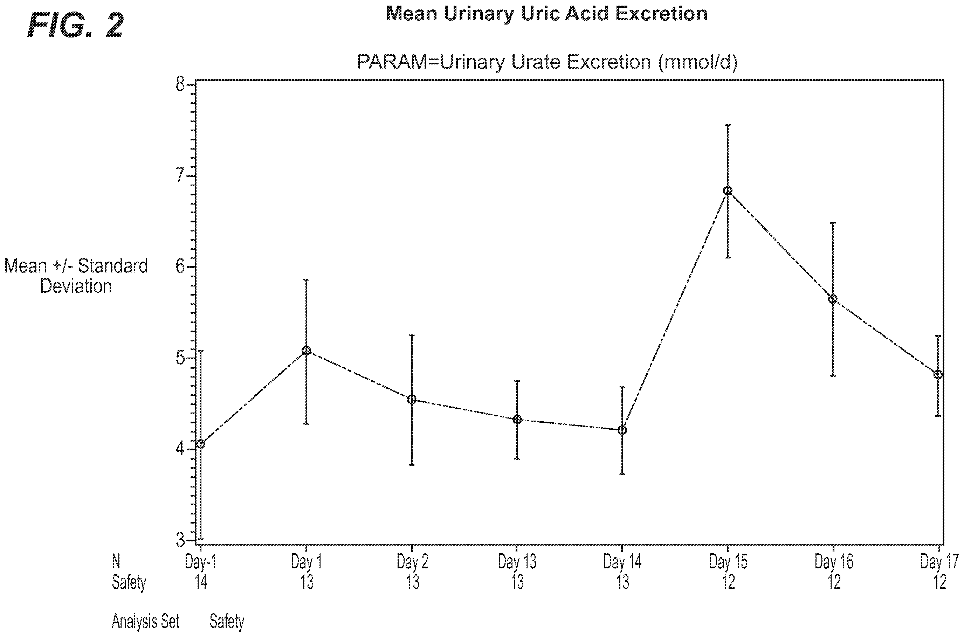

Table 2, below, lists mean urine uric acid excretion and calculated mean changes through the course of the study. Figure 2 illustrates said mean uric acid excretion (± standard deviation) as a function of study day.

**Table 2: Urine Urate Excretion (mmole/day)- 12 patients**

| | **Mean (mmol/d)** | **Mean Change from Day-1 (µmol/d)** |
|---|---|---|
| **Day -1** | 4.047 | - |
| **Day 1** | 5.068 | 0.796 |
| **Day 2** | 4.544 | 0.273 |
| **Day 13** | 4.319 | 0.048 |
| **Day 14** | 4.208 | -0.064 |
| **Day 15** | 6.836 | 2.633 |
| **Day 16** | 5.642 | 1.440 |
| **Day 17** | 4.807 | 0.605 |

Mean urinary urate excretion (mmol/day) increased compared to baseline on Days 1, 2, and 13 (by approximately 19%, 6%, and 1%, respectively) during treatment with canagliflozin alone. Upon initiating probenecid dosing together with canagliflozin on Day 15, mean urinary urate excretion increased (by approximately 63% from baseline), and then declined towards baseline levels on Day 16 and Day 17 during continued probenecid co-administration (to 34% and 14% increase from baseline).

These study results indicate serum urate levels decreased on average by about 19% to 24% compared to baseline, during the initial 14-day canagliflozin treatment. When probenecid was co-administered with canagliflozin, further reduction of serum urate was observed, and there was a corresponding transient augmentation of 24-hour urate excretion in the urine. These data indicate that co-administration of canagliflozin with probenecid augments serum urate lowering observed with canagliflozin alone through a uricosuric mechanism.

## Claims

1. Canagliflozin for use in a method for treating hyperuricemia or a related disorder, wherein the method comprises administering to a subject in need thereof, a therapeutically effective amount of co-therapy comprising canagliflozin and probenecid, wherein the term "hyperuricemia or a related disorder" is defined as any disease, disorder or condition **characterized by** elevated, i.e. above normal, levels serum uric acid.

2. Probenecid for use in a method for treating hyperuricemia or a related disorder, wherein the method comprises administering to a subject in need thereof, a therapeutically effective amount of co-therapy comprising canaglifozin and probenecid, wherein the term "hyperuricemia or a related disorder" is defined as any disease, disorder or condition **characterized by** elevated, i.e. above normal, levels serum uric acid.

3. Canagliflozin and probenecid for use in a method for treating hyperuricemia or a related disorder, wherein the method comprises administering to a subject in need thereof, a therapeutically effective amount of co-therapy comprising canaglifozin and probenecid, wherein the term "hyperuricemia or a related disorder" is defined as any disease, disorder or condition **characterized by** elevated, i.e. above normal, levels serum uric acid.

4. The compound or compounds of any of claims 1 to 3 for use as in any of claims 1 to 3, wherein the treatment of hyperuricemia or related disorder prevents symptoms of gout.

5. The compound or compounds of any of claims 1 to 3 for use as in any of claims 1 to 3, wherein the hyperuricemia or related disorder is selected from the group consisting of gout, urate nephropathy, chronic kidney disease, hypertension due to hyperurecemia, and kidney stones.

6. The compound or compounds of any of claims 1 to 3 for use as in any of claims 1 to 3, wherein the canagliflozin is present as a crystalline hemihydrate.

7. The compound or compounds of any of claims 1 to 3 for use as in any of claims 1 to 3, wherein the canagliflozin is administered in an amount in the range of from about 50 to about 500 mg, or wherein the canagliflozin is administered in an amount in the range of from about 100 to about 300 mg, or wherein the probenecid is administered in an amount in the range of from about 250 to about 1000 mg.

8. The compound or compounds of any of claims 1 to 3 for use as in any of claims 1 to 3, wherein the canagliflozin is administered in an amount in the range of from about 100 mg to about 300 mg per day; and wherein the probenecid is administered in an amount in the range of from about 250 mg to about 1000 mg per day.

9. Canagliflozin for use in a method for treating gout, wherein the method comprises administering to a subject in need thereof, a therapeutically effective amount of co-therapy comprising canagliflozin and probenecid.

10. Probenecid for use in a method for treating gout, wherein the method comprises administering to a subject in need thereof, a therapeutically effective amount of co-therapy comprising canaglifozin and probenecid.

11. Canagliflozin and probenecid for use in a method for treating gout, wherein the method comprises administering to a subject in need thereof, a therapeutically effective amount of co-therapy comprising canaglifozin and probenecid.

12. A pharmaceutical composition comprising a therapeutically effective amount co-therapy of canagliflozin and probenecid; and a pharmaceutically acceptable carrier.

13. A pharmaceutical composition as in Claim 12, wherein the canagliflozin is present as a crystalline hemihydrate.

14. A pharmaceutical composition as in Claim 12, wherein the canagliflozin is in an amount in the range of from about 50 to about 500 mg, or wherein the canagliflozin is an amount in the range of from about 100 to about 300 mg, or wherein the probenecid is in an amount in the range of from about 250 to about 1000 mg.

15. A pharmaceutical composition as in Claim 12, wherein the canagliflozin is in an amount in the range of from about 100 mg to about 300 mg per day; and wherein the probenecid is administered in an amount in the range of from about 250 mg to about 1000 mg per day.

## Patentansprüche

1. Canagliflozin zum Gebrauch in einem Verfahren zum Behandeln von Hyperurikämie oder einer verwandten Erkrankung, wobei das Verfahren das Verabreichen einer therapeutisch wirksamen Menge Kombinationstherapie umfassend Canagliflozin und Probenecid an einen Patienten mit Bedarf daran umfasst, wobei die Bezeichnung "Hyperurikämie oder eine verwandte Erkrankung" definiert ist als jedwede Krankheit, Störung oder Beschwerde, die durch erhöhte, d. h. oberhalb des normalen Bereichs liegende, Serum-Harnsäure-Konzentrationen gekennzeichnet ist.

2. Probenecid zum Gebrauch in einem Verfahren zur Behandlung von Hyperurikämie oder einer verwandten Erkrankung, wobei das Verfahren das Verabreichen einer therapeutisch wirksamen Menge Kombinationstherapie umfassend Canagliflozin und Probenecid an einen Patienten mit Bedarf daran umfasst, wobei die Bezeichnung "Hyperurikämie oder eine verwandte Erkrankung" definiert ist als jedwede Krankheit, Störung oder Beschwerde, die durch erhöhte, d. h. oberhalb des normalen Bereichs liegende, Serum-Harnsäure-Konzentrationen gekennzeichnet ist.

3. Canagliflozin und Probenecid zum Gebrauch in einem Verfahren zur Behandlung von Hyperurikämie oder einer verwandten Erkrankung, wobei das Verfahren das Verabreichen einer therapeutisch wirksamen Menge Kombinationstherapie umfassend Canagliflozin und Probenecid an einen Patienten mit Bedarf daran umfasst, wobei die Bezeichnung "Hyperurikämie oder eine verwandte Erkrankung" definiert ist als jedwede Krankheit, Störung oder Beschwerde, die durch erhöhte, d. h. oberhalb des normalen Bereichs liegende, Serum-Harnsäure-Konzentrationen gekennzeichnet ist.

4. Verbindung oder Verbindungen nach einem der Ansprüche 1 bis 3 zum Gebrauch wie nach einem der Ansprüche 1 bis 3, wobei die Behandlung von Hyperurikämie oder einer verwandten Erkrankung Gichtsymptomen vorbeugt.

5. Verbindung oder Verbindungen nach einem der Ansprüche 1 bis 3 zum Gebrauch wie in einem der Ansprüche 1 bis 3, wobei die Hyperurikämie oder verwandte Erkrankung ausgewählt ist aus der Gruppe bestehend aus Gicht, Uratnephropathie, chronischer Nierenerkrankung, Bluthochdruck durch Hyperurikämie und Nierensteinen.

6. Verbindung oder Verbindungen nach einem der Ansprüche 1 bis 3 zum Gebrauch wie in einem der Ansprüche 1 bis 3, wobei das Canagliflozin als ein kristallines Halbhydrat vorhanden ist.

7. Verbindung oder Verbindungen nach einem der Ansprüche 1 bis 3 zum Gebrauch wie in einem der Ansprüche 1 bis 3, wobei das Canagliflozin in einer Menge im Bereich von etwa 50 bis etwa 500 mg verabreicht wird, oder wobei das Canagliflozin in einer Menge im Bereich von etwa 100 bis etwa 300 mg verabreicht wird, oder wobei das Probenecid in einer Menge im Bereich von etwa 250 bis etwa 1000 mg verabreicht wird.

8. Verbindung oder Verbindungen nach einem der Ansprüche 1 bis 3 zum Gebrauch wie in einem der Ansprüche 1 bis 3, wobei das Canagliflozin in einer Menge im Bereich von etwa 100 mg bis etwa 300 mg pro Tag verabreicht wird; und wobei das Probenecid in einer Menge im Bereich von etwa 250 mg bis etwa 1000 mg pro Tag verabreicht wird.

9. Canagliflozin zum Gebrauch in einem Verfahren zur Behandlung von Gicht, wobei das Verfahren das Verabreichen einer therapeutisch wirksamen Menge Kombinationstherapie umfassend Canagliflozin und Probenecid an einen Patienten mit Bedarf daran umfasst.

10. Probenecid zum Gebrauch in einem Verfahren zur Behandlung von Gicht, wobei das Verfahren das Verabreichen einer therapeutisch wirksamen Menge Kombinationstherapie umfassend Canagliflozin und Probenecid an einen Patienten mit Bedarf daran umfasst.

11. Canagliflozin und Probenecid zum Gebrauch in einem Verfahren zur Behandlung von Gicht, wobei das Verfahren das Verabreichen einer therapeutisch wirksamen Menge Kombinationstherapie umfassend Canagliflozin und Probenecid an einen Patienten mit Bedarf daran umfasst.

12. Pharmazeutische Zusammensetzung, die eine therapeutisch wirksame Menge Kombinationstherapie von Canagliflozin und Probenecid und einen pharmazeutisch akzeptablen Träger umfasst.

13. Pharmazeutische Zusammensetzung wie in Anspruch 12, wobei das Canagliflozin als ein kristallines Halbhydrat vorhanden ist.

14. Pharmazeutische Zusammensetzung wie in Anspruch 12, wobei das Canagliflozin in einer Menge im Bereich von etwa 50 bis etwa 500 mg vorhanden ist, oder wobei das Canagliflozin in einer Menge im Bereich von etwa 100 bis etwa 300 mg vorhanden ist, oder wobei das Probenecid in einer Menge im Bereich von etwa 250 bis etwa 1000 mg vorhanden ist.

15. Pharmazeutische Zusammensetzung wie in Anspruch 12, wobei das Canagliflozin in einer Menge im Bereich von etwa 100 mg bis etwa 300 mg pro Tag verabreicht wird; und wobei das Probenecid in einer Menge im Bereich von etwa 250 mg bis etwa 1000 mg pro Tag verabreicht wird.

## Revendications

1. Canagliflozine pour utilisation dans une méthode de traitement de l'hyperuricémie ou d'un trouble apparenté, où la méthode comprend l'administration à un sujet le nécessitant d'une quantité thérapeutiquement active d'une thérapie combinatoire comprenant la canaglifozine et le probénécid, où l'expression « hyperuricémie ou trouble apparenté » est définie comme étant n'importe quelle maladie, trouble ou état pathologique **caractérisés par** des niveaux élevés, c'est-à-dire supérieurs à la normale, d'acide urique sérique.

2. Probénécid pour utilisation dans une méthode de traitement de l'hyperuricémie ou d'un trouble apparenté, où la méthode comprend l'administration à un sujet le nécessitant d'une quantité thérapeutiquement active d'une thérapie combinatoire comprenant la canaglifozine et le probénécid, où l'expression « hyperuricémie ou trouble apparenté » est définie comme étant n'importe quelle maladie, trouble ou état pathologique **caractérisés par** des niveaux élevés, c'est-à-dire supérieurs à la normale, d'acide urique sérique.

3. Canagliflozine et probénécid pour utilisation dans une méthode de traitement de l'hyperuricémie ou d'un trouble apparenté, où la méthode comprend l'administration à un sujet le nécessitant d'une quantité thérapeutiquement active d'une thérapie combinatoire comprenant la canaglifozine et le probénécid, où l'expression « hyperuricémie ou trouble apparenté » est définie comme étant n'importe quelle maladie, trouble ou état pathologique **caractérisés par** des niveaux élevés, c'est-à-dire supérieurs à la normale, d'acide urique sérique.

4. Composé ou composés selon l'une quelconque des revendications 1 à 3 pour utilisation selon l'une quelconque des revendications 1 à 3, où le traitement de l'hyperuricémie ou d'un trouble apparenté prévient les symptômes de la goutte.

5. Composé ou composés selon l'une quelconque des revendications 1 à 3 pour utilisation dans l'une quelconque des revendications 1 à 3, où l'hyperuricémie ou le trouble apparenté sont choisis dans le groupe constitué par les suivants : goutte, néphropathie uratique, maladie rénale chronique, hypertension due à une hyperuricémie, et calculs rénaux.

6. Composé ou composés selon l'une quelconque des revendications 1 à 3 pour utilisation selon l'une quelconque des revendications 1 à 3, où la canagliflozine est présente sous forme d'un hémihydrate cristallin.

7. Composé ou composés selon l'une quelconque des revendications 1 à 3 pour utilisation selon l'une quelconque des revendications 1 à 3, où la canagliflozine est administrée à une teneur comprise dans la plage allant d'environ 50 à environ 500 mg, ou où la canagliflozine est administrée à une teneur comprise dans la plage allant d'environ 100 à environ 300 mg, ou où le probénécid est administré à une teneur comprise dans la plage allant d'environ 250 à environ 1000 mg.

8. Composé ou composés selon l'une quelconque des revendications 1 à 3 pour utilisation selon l'une quelconque des revendications 1 à 3, où la canagliflozine est administrée à une teneur comprise dans la plage allant d'environ 100 mg à environ 300 mg par jour ; et où le probénécid est administré à une teneur comprise dans la plage allant d'environ 250 mg à environ 1000 mg par jour.

9. Canagliflozine pour utilisation dans une méthode de traitement de la goutte, où la méthode comprend l'administration à un sujet le nécessitant d'une quantité thérapeutiquement active d'une thérapie combinatoire comprenant la canaglifozine et le probénécid.

10. Probénécid pour utilisation dans une méthode de traitement de la goutte, où la méthode comprend l'administration à un sujet le nécessitant d'une quantité thérapeutiquement active d'une thérapie combinatoire comprenant la canaglifozine et le probénécid.

11. Canagliflozine et probénécid pour utilisation dans une méthode de traitement de la goutte, où la méthode comprend l'administration à un sujet le nécessitant d'une quantité thérapeutiquement active d'une thérapie combinatoire comprenant la canaglifozine et le probénécid.

12. Composition pharmaceutique comprenant une quantité thérapeutiquement active d'une thérapie combinatoire de canagliflozine et de probénécid ; et un vecteur pharmaceutiquement acceptable.

13. Composition pharmaceutique selon la Revendication 12, où la canagliflozine est présente sous forme d'un hémihydrate cristallin.

14. Composition pharmaceutique selon la Revendication 12, où la canagliflozine est présente à une teneur comprise dans la plage allant d'environ 50 à environ 500 mg, ou où la canaglifozine est présente à une teneur comprise dans la plage allant d'environ 100 à environ 300 mg, ou où le probénécid est présent à une teneur comprise dans la plage allant d'environ 250 à environ 1000 mg.

15. Composition pharmaceutique selon la Revendication 12, où la canaglifozine est présente à une teneur comprise dans la plage allant d'environ 100 mg à environ 300 mg par jour ; et où le probénécid est administré à une teneur comprise entre environ 250 mg et environ 1000 mg par jour.
